# EUROPEAN PATENT SPECIFICATION

(11) **EP 1 773 292 B1**
(45) Date of publication and mention of the grant of the patent: **11.03.2009**
(21) Application number: 05757758.7
(22) Date of filing: 30.06.2005
(51) Int. Cl.: A61K 9/20

(54) **RAPIDLY DISINTEGRATING ORODISPERSIBLE COMPOSITION CONTAINING NONFILAMENTOUS COPROCESSED POLYOLS PARTICLES AND SILICIFIED MICROCRYSTALLINE CELLULOSE**
SCHNELL ZERFALLENDE IM MUND DISPERGIERBARE ZUSAMMENSETZUNG MIT NICHT-FILAMENTÖSEN MITVERARBEITETEN POLYOL-TEILCHEN UND VERKIESELTER MIKROKRISTALLINER CELLULOSE
COMPOSITION ORODISPERSIBLE A DESINTEGRATION RAPIDE CONTENANT DES PARTICULES NON FILAMENTEUSES A BASE DE POLYOLS COTRAITES ET DE LA CELLULOSE MICROCRISTALLINE SILICIFIEE

(30) Priority: 01.07.2004 SI 200400199
(43) Date of publication of application: 18.04.2007
(73) Proprietor: LEK Pharmaceuticals d.d., 1526 Ljubljana (SI)
(72) Inventor: BESO, Adnan, 8000 Novo Mesto (SI); SIRCA, Judita, 1000 Ljubljana (SI)
(74) Representative: Kröger, Bernd
(86) International application number: PCT/EP2005/007091
(87) International publication number: WO 2006/002937

(56) References cited:
- WO-A-96/21429
- WO-A-03/051338
- ZELEZNIK ET AL: "High Functionality Excipients (HFE)- Prosolv SMCC as an Effective Strategy for Generic Drug Formulation"[Online] January 2004 (2004-01), pages 1-4, XP002337089 Retrieved from the Internet: URL:http://www.jrspharma.com/pubs/prosolv_ effective_strategy.pdf>
- LAHDENPAA E ET AL: "DIRECT COMPRESSION WITH SILICIFIED AND NON-SILICIFIED MICROCRYSTALLINE CELLULOSE: STUDY OF SOME PROPERTIES OF POWDERS AND TABLETS" SCIENCES TECHNIQUES ET PRATIQUES STP PHARMA SCIENCES, PARIS, FR, vol. 11, no. 2, March 2001 (2001-03), pages 129-135, XP009006009 ISSN: 1157-1489
- RIBA M D ET AL: "SILICIFIED MICROCRYSTALLINE CELLULOSE: A COMPARATIVE EVALUATION OF THIS NEW HIGH FUNCTIONALITY DIRECT COMPRESSION EXCIPIENT" PHARMACEUTICAL RESEARCH, NEW YORK, NY, US, vol. 14, no. 11, SUPPL, November 1997 (1997-11), page S11, XP009007897 ISSN: 0724-8741
- NACHAEGARI S K ET AL: "Coprocessed Excipients for Solid Dosage Forms" PHARMACEUTICAL TECHNOLOGY, EUGENE, OR, US, vol. 28, no. 1, January 2004 (2004-01), pages 52-64, XP002337087
- "Promosolv SMCC 90" JRS PHARMA LP, [Online] April 2003 (2003-04), XP002341967 Retrieved from the Internet: URL:http://www.jrspharma.com/prosolv_pdfs/ prosolv_smcc_90_rev_02.pdf> [retrieved on 2005-08-23]
- EDGE S ET AL: "The location of silicon dioxide in silicified microcrystalline cellulose" PHARMACY AND PHARMACOLOGY COMMUNICATIONS 1999 UNITED KINGDOM, vol. 5, no. 6, 1999, pages 371-376, XP009052929 ISSN: 1460-8081

## Description

### Field of the invention

The present invention belongs to the field of the pharmaceutical technology and it relates to rapidly disintegrating pharmaceutical compositions intended to be used as orodispersible tablets.

### Background of the invention

Application of solid dosage forms may present a reasonable difficulty or impracticality for many patients. Particularly pediatric and geriatric patients find difficulties with swallowing or chewing solid dosage forms. Producing more patient-friendly dosage forms that rapidly disintegrate in the mouth can improve patients compliance.

Rapidly disintegrating solid dosage forms are becoming used more and more frequently because administration of these dosage forms is not an issue for patients having problems with swallowing. As this dosage form disintegrates in the mouth within seconds after application, leaving a dispersion on the tongue that is easily swallowed with saliva, there is no need to administer with a drink. Because of this, patients' compliance improves, making pharmacotherapy more efficient.

Different techniques for preparation of rapidly disintegration tablets are described in the patent literature.

EP 0 494 972 B1 describes a solid dosage form adapted for direct oral administration comprising a water or saliva activated effervescent disintegration agent and a plurality of microcapsules, said microcapsules including a systematically distributable pharmaceutical ingredient.

US 5,464,632 relates to a rapidly disintegrable multiparticulate tablet comprising active substance in the form of coated or uncoated microcrystals or microgranules and a mixture of excipients, The mixture of excipients is responsible for the disintegration and comprises a disintegrating agent and a swelling agent.

US 5,501,861 and US 5,720,974 disclose that intrabuccally dissolving tablets having an adequate strength and a porous structure which quickly disintegrate and dissolve in the buccal cavity can be obtained by preparing a mixture of an active ingredient, a saccharide and water in a sufficient amount to wet the surface of the saccharide granules, tabletting the mixture into tablets and drying the tablets.

WO 93/15724 disclose production methods of tablets wherein tablets composed of saccharide are produced in such a manner that saccharide mixture supplied with appropriate water is compressed at a low pressure and then dried by freeze-drying or vacuum drying to make solid tablets.

EP 0 636 364 B1 describes a rapidly disintegrating dosage form comprising a coated particle comprising pharmaceutical particles coated with a taste-masking coating, a water-disintegrable, compressible carbohydrate and a binder.

Rapidly disintegrating freeze-dried dosage forms are disclosed in US 5,631,023 and US 5,976,577.

EP 0 745 382 B1 is directed to intrabuccally quick disintegrating and dissolving compressed molding, which comprises granules comprising a saccharide having low moldability having been coated and/or granulated with a saccharide having high moldability as a binder.

US 2001/0014340 discloses an intrabuccally rapidly disintegrating tablet comprising sugar alcohol or saccharide having an average particle diameter of not more than 30 µm, an active ingredient, and a disintegrant.

EP 0 839 526 A2 describes a solid pharmaceutical preparation with fast buccal disintegration or dissolution comprising a pharmaceutically active ingredient, erythritol, crystalline cellulose and a disintegrant.

US 6,149,938 describes a process for making a granulate composition suitable for the preparation of mouth-soluble, rapidly disintegrating tablets, which comprises granulating a polyalcohol, an active ingredient, edible acid, other excipients, an aqueous solution or dispersion of a water-soluble or water-dispersible polymer, and drying the granulate.

WO 99/47126 relates to rapidly dissolving compressed tablets comprising a non-saccharide water soluble polymer, which are free of organic solvent residues, and a method of production thereof.

US 6,024,981 discloses a rapidly dissolvable dosage form adapted for direct oral dosing comprising an active ingredient and a matrix of a non-direct compression filler and a relatively high lubricant content.

WO 00/06126 describes a rapidly disintegrable solid preparation comprising a pharmaceutically active ingredient, sugar and a low-substituted hydroxypropylcellulose.

WO 00/09090 relates to an orally rapidly disintegrating tablet comprising an active ingredient in the form of a powder or microcapsule, a filler and an in-mouth viscosity enhancing material, which provide an organoleptically pleasant viscous slurry upon disintegration in the mouth.

WO 00/57857 discloses a rapidly disintegrable tablet for oral administration comprising pharmacologically active ingredient, spray-dried mannitol as a disintegrant, crospovidone as a co-disintegrant and one or more pharmaceutically acceptable excipients, without microcrystalline cellulose.

EP 1 203 580 relates to rapidly disintegrating solid preparations, which contain an active ingredient, D-mannitol having an average particle size between 30 µm and 300 µm, a disintegrant and celluloses.

EP 1 260 215 A1 describes a rapidly disintegrating tablet containing an active ingredient, a saccharide and a polyvinyl alcohol.

EP 1 295 595 A1 is directed to a tablet which, rapidly disintegrates in the buccal cavity comprising spray-dried drug-containing particles and a saccharide. The particles consist of a bitter tasting drug and/or drug of inferior fluidity and a pharmaceutical preparation carrier, which is a water-insoluble polymer. The saccharide is a granulation product obtained by spraying to coat and/or granulate a saccharide having low moldability using a saccharide of high moldability as a binder.

WO 03/051338 discloses a co-processed carbohydrate system that produces formulations that are directly compressible into solid dosage forms, which rapidly and completely dissolve and/or disintegrate in the oral cavity within 60 seconds.

WO 96/21429 discloses a novel pharmaceutical excipient - silicified microcrystalline cellulose. Properties of silicified microcrystalline cellulose and benefits of using it are described in several articles:
- Zeleznik J.A. et al.: "High Functionality Excipients (HFE) - Prosolv SMCC as an Effective Strategy for Generic Drug Formulation"[Online] January 2004, pages 1-4, Retrieved from the Internet: URL:http://www.jrspharma.com/pubs/prosolv_ effective_strategy.pdf>
- Lahdenpaa E. et al.: "Direct compression with silicified and non-silicified microcrystalline cellulose: study of some properties of powders and tablets" Scieces techniques et pratiques S.T.P. Pharma Sciences, Paris, FR, vol. 11, no. 2, March 2001, pages 129-135
- Riba M.D. et al.: "Silicified microcrystalline cellulose: A comparative evaluation of this new high functionality direct compression excipient" Pharmaceutical Research, New York, NY, US, vol. 14, no. 11, SUPPL, November 1997, page S11
- Nachaegari S.K. et al.: "Coprocessed Excipients for Solid Dosage Forms" Pharmaceutical Technology, Eugene, OR, US, vol. 28, no. 1, January 2004, pages 52-64
- "Prosolv SMCC 90" JRS Parma LP, [Online] April 2003, Retrieved from the Internet:
   URL:http://www.jrspharma.com/prosolv_pdfs/prosolv_smcc_90_rev_02.pdf> [retrieved on 2005-08-23]
- Edge S. Et al.: "The location of silicon dioxide in silicifed microcrystalline cellulose" Pharmacy and Pharmacology Communications, United Kingdom, vol. 5, no. 6, 1999, pages 371-376

The aim of the present invention is to provide a patient-friendly orodispersible pharmaceutical composition that may be taken without water, leaving a pleasant mouth sensation and hiding or diminishing any unpleasant taste.

The pharmaceutical composition of the present invention is made by a simple manufacturing process using conventional processing equipment.

Additionally, the pharmaceutical composition of the present invention is suitable for packaging on a conventional packaging line.

### Summary of the invention

The present invention relates to novel rapidly disintegrating pharmaceutical compositions useful as orodispersible tablets. The composition of the present invention comprises:
- granules comprising at least one active substance,
- a fast dissolving excipient based on co-processed saccharides or polyols, wherein the excipient particles have a nonfilamentous microstructure,
- silicified microcrystalline cellulose, and
- optional excipients.

In another embodiment, the present invention relates to novel rapidly disintegrating pharmaceutical compositions useful as orodispersible tablets. The composition of the present invention comprises:
- granules comprising at least one active substance,
- a fast dissolving excipient based on co-processed mannitol and sorbitol, wherein the excipient particles have a nonfilamentous microstructure,
- silicified microcrystalline cellulose, and
- optional excipients.

In another embodiment, the present invention relates to novel rapidly disintegrating pharmaceutical compositions useful as orodispersible tablets. The composition of the present invention comprises:
- granules comprising at least one active substance which needs to be granulated before compressing into a pharmaceutical composition,
- a fast dissolving excipient based on co-processed saccharides or polyols, wherein the excipient particles have a nonfilamentous microstructure,
- silicified microcrystalline cellulose, and
- optional excipients.

In another embodiment, the present invention relates to novel rapidly disintegrating pharmaceutical compositions useful as orodispersible tablets. The composition of the present invention comprises:
- water soluble granules comprising at least one poorly soluble and/or cohesive active substance which needs to be granulated before compressing into a pharmaceutical composition,
- a fast dissolving excipient based on co-processed saccharides or polyols, wherein the excipient particles have a nonfilamentous microstructure,
- silicified microcrystalline cellulose, and
- optional excipients.

In another embodiment, the present invention relates to novel rapidly disintegrating pharmaceutical compositions useful as orodispersible tablets. The composition of the present invention comprises:
- water soluble granules comprising at least one antihistaminic agent,
- a fast dissolving excipient based on co-processed saccharides or polyols, wherein the excipient particles have a nonfilamentous microstructure,
- silicified microcrystalline cellulose, and
- optional excipients.

In another embodiment, the present invention relates to novel rapidly disintegrating pharmaceutical compositions useful as orodispersible tablets. The composition of the present invention comprises:
- water soluble granules comprising at least loratadine,
- a fast dissolving excipient based on co-processed saccharides or polyols, wherein the excipient particles have a nonfilamentous microstructure,
- silicified microcrystalline cellulose, and
- optional excipients.

In another embodiment, the present invention relates to a process for the preparation of pharmaceutical composition of the present invention comprising:
- preparing granules comprising at least one active substance which needs to be granulated before compressing into a pharmaceutical composition,
- blending the granules with a fast dissolving excipient based on co-processed saccharides or polyols, silicified microcrystalline cellulose and optional excipients,
- compressing the resulting mixture into tablet form.

In another embodiment, the present invention relates to a process for the preparation of pharmaceutical composition of the present invention comprising:
- preparing granules comprising at least one active substance which needs to be granulated before compressing into a pharmaceutical composition,
- coating the granules,
- blending the coated granules with a fast dissolving excipient based on co-processed saccharides or polyols, silicified microcrystalline cellulose and optional excipients,
- compressing the resulting mixture into tablet form.

### Detailed description of the invention

Some of the prior art techniques for preparing rapidly disintegrating tablets are generally technologically very demanding and require expensive manufacturing equipment. However, new commercially available excipients, e.g. the co-processed carbohydrate system, have enabled production of rapidly disintegrating tablets using conventional processing equipment and procedures.

In the process of development of a loratadine-based rapidly disintegrating pharmaceutical composition we used a fast dissolving excipient based on co-processed mannitol and sorbitol.

An example of fast dissolving excipient based on co-processed mannitol and sorbitol, wherein the excipient particles have a nonfilamentous microstructure is described in WO 03/051338, included herein by reference. Described excipient is directly compressible into solid dosage forms.

When a powder blend of the fast dissolving excipient based on co-processed mannitol and sorbitol comprising particles having a nonfilamentous microstructure with loratadine was made it was found that loratadine, which is a highly cohesive substance, made the otherwise superior flow properties of said excipient poor. Furthermore, we faced a problem of powder sticking to the surface of the tabletting tool. Addition of acceptable amounts of lubricants improved the flowability reasonably, however the problem of dye sticking remained. Both flowability and tabletting problems were resolved by granulating the active substance.

Upon blending granulate containing loratadine with the fast dissolving excipient based on co-processed mannitol and sorbitol comprising particles having a nonfilamentous microstructure we faced a new processing problem, namely segregation. It was found that the segregation occurred mostly due to the great difference in particle sizes of granulated active substance and said excipient.

In order to reduce the particle size difference between the granulate and the fast dissolving excipient based on co-processed mannitol and sorbitol comprising particles having a nonfilamentous microstructure, we first tried to reduce granulate particle size by grinding. However due to the cohesiveness of the active substance it was practically impossible to produce sufficiently small particles to avoid or reduce segregation trend. Furthermore, by reducing the particle size of granulate containing loratadine the problem of sticking becomes apparent once again. This is a result of more surface of the active substance becoming available for interactions with the surroundings compared to the granulated active substance.

The appropriate solution for the segregation issue was gained by replacing a part of the fast dissolving excipient based on co-processed mannitol and sorbitol comprising particles having a nonfilamentous microstructure with silicified microcrystalline cellulose (SMCC). Due to its large specific surface area SMCC can adsorb a sufficient quantity of fine dust of the fast dissolving excipient based on co-processed mannitol and sorbitol comprising particles having a nonfilamentous microstructure, to reduce and prevent the segregation. Due to the irregular shapes of SMCC particles a free flow of formulation components is limited, which further preserves the homogeneity within the tabletting mass, achieved in the process of blending. When SMCC is used alone in share higher than 50% w/w it leaves an unpleasant "gritty" sensation in the mouth, as SMCC does not dissolve in saliva. Tablets containing 40 - 60% of the fast dissolving excipient based on co-processed mannitol and sorbitol comprising particles having a nonfilamentous microstructure and 10 - 30% SMCC show no segregation trend and have no gritty sensation as a relatively small amount of SMCC is used in preparing the formulation. Silicified microcrystalline cellulose may be any commercially available form of this excipient, for example Prosolv SMCC, described in WO 96/21429, included herein by reference.

Fast disintegrating tablets produced using conventional tabletting tools need to have significantly lower tensile strength then usual tablets. However they still need to comply with the friability standards in order to assure the patient receives the intact tablet. The pharmaceutical composition with the combination of excipients as just described offers superior characteristics in this respect as well. Tablets made of the fast dissolving excipient based on co-processed mannitol and sorbitol comprising particles having a nonfilamentous microstructure:SMCC combination can be produced with lower tensile strength and have at the same time lower friability than tablets formulated with the fast dissolving excipient based on co-processed mannitol and sorbitol comprising particles having a nonfilamentous microstructure alone. This feature is important for fast disintegration/dissolution and enabling simple packaging of the tablet. The SMCC component assures a better disintegration and improved dissolution characteristics compared to the single excipient pharmaceutical composition. Furthermore, it improves the compressibility of the tabletting mass.

While the fast dissolving excipient based on co-processed mannitol and sorbitol comprising particles having a nonfilamentous microstructure itself contains above all a high share of polyols that in general have the characteristics of making solid dosage form gradually harder over time, an addition of SMCC can prevent potential instability of the dosage form.

Therefore in a first embodiment, the present invention relates to rapidly disintegrating pharmaceutical compositions intended to be used as orodispersible tablets comprising granules comprising active substance, a fast dissolving excipient based on co-processed saccharides or polyols having a nonfilamentous microstructure, silicified microcrystalline cellulose and optionally one or more pharmaceutically acceptable excipients.

The "rapidly disintegrating pharmaceutical composition" in the present invention means a pharmaceutical composition that disintegrates preferably within 60 seconds after contact with water or saliva.

A "fast dissolving excipient" as referred to herein means an excipient, which allows rapid disintegration of a pharmaceutical composition, preferably within 60 seconds, after contact with water or saliva due to its high and rapid solubility in water.

The "orodispersible tablet" in the present invention means a tablet that is capable of disintegrating in the buccal cavity by contact with saliva only without additionally taking water.

The "co-processed saccharides or polyols" in the present invention means the excipient produced by drying a solution of at least two saccharides or polyols in an air stream to make a single product.

The pharmaceutical composition of the present invention is especially appropriate for an active substance, which needs to be granulated before compressing into pharmaceutical compositions due to their biopharmaceutical or physico-chemical properties. Advantageously the active substance is a substance, which has to be administered in low dosage or has unfavourable physical or chemical characteristics, e.g. cohesiveness, or unpleasant taste.

The pharmaceutical composition of the present invention may be also used for administration of an active substance, which may be included in modified release microparticles, e.g. extended, controlled or targeted release microparticles, to provide appropriate administration of the active substance.

Active substance may be selected from the group consisting of pharmaceuticals, vitamins, minerals, dietary supplements, vegetable extracts and mixtures thereof.

Examples of pharmaceuticals that may be used include, but are not limited to gastrointestinal function conditioning agents, anti-inflammatory agents, analgesics, agents for erectile dysfunction therapy, anti-depressants, sedatives, hypnotics, neuroleptics, anti-migraines, antihistaminic agents, for example loratadine, desloratadine, pseudoephedrine, cetirizine and mixture thereof, anti-bacterial agents, antiviral agents, cardiovascular agents, diuretics, anti-hypertensive agents anti-hypolipidemic agents, anti-ulcer agents, antiemetics, anti-asthmatic agents, anti-depressants, anti-thrombotic agents, chemotherapeutic agents, hormones, anti-helmintic agents, anti-diabetic agents, corticosteroids, peptides, proteins, recombinant drugs and mixtures thereof.

The granules comprising active substance may be uncoated or coated and may be prepared by any method known in the field of pharmaceutical technology, e.g. dry granulation or wet granulation methods, and may comprise any known acceptable pharmaceutical excipients convenient for preparation of granules.

The granules may be coated with any coating materials known in the field of pharmaceutical technology, e.g. gastrosoluble polymers, enterosoluble polymers, and the coating may be applied to the granules by any process known in the field of the pharmaceutical technology, e.g. spraying the coating dispersion onto the granules.

The pharmaceutical composition of the invention comprises a fast dissolving excipient based on co-processed saccharides or polyols having a nonfilamentous microstructure. Saccharides may be selected from the group consisting of lactose, fructose, dextrose, sucrose, maltose, and their derivates such as maltitol, lactitol, isomalt and mixtures thereof.

Polyols, which are sugar alcohols of the general formula CH₂OH-(CHOH)*ₙ-*CH₂OH, wherein n is 2 to 6, and their dimeric anhydrides, may be preferably selected from the group consisting of erythritol, mannitol, sorbitol, xylitol, and mixtures thereof.

Preferably the fast dissolving excipient based on co-processed saccharides or polyols is the fast dissolving excipient based on co-processed mannitol and sorbitol, most preferably the fast dissolving excipient is based on co-spray-dried mannitol and sorbitol.

The pharmaceutically acceptable excipients may be selected from the group consisting of acidifying agents, sweetening agents, flavourings, anticaking agents, glidants, disintegrants, lubricants and other excipients known in the art.

Acidifying agents may be selected form the group consisting of citric acid, lactic acid, and tartaric acid. Preferably the acidifying agent is citric acid.

Sweetening agents may be selected form the group consisting of aspartame, acesulfames, cyclamates, and saccharin. Preferably the sweetening agent is aspartame.

Glidants may be selected form the group consisting of colloidal silicone dioxide, talc, magnesium trisilicate, cellulose, starch, magnesium, calcium or aluminium stearate, stearic acid, palmitic acid, cetanol, stearol (1-octadecanol), etc. Preferably the glidant is colloidal silicon dioxide.

Lubricants may be selected form the group consisting of magnesium, calcium or aluminium stearate, glyceryl monostearates, glycerin palmitostearate, stearic acid, sodium stearyl fumarate, fumaric acid, polyethylene glycols of various molecular weights, siliconized talc, cutina HR, glyceryl behenate, sodium benzoate, and hydrogenated castor oil. Preferably the lubricant is magnesium stearate.

In another embodiment, the present invention relates to rapidly disintegrating pharmaceutical compositions intended to be used as orodispersible tablets comprising water soluble granules comprising a poorly soluble and/or cohesive active substance, preferably loratadine or desloratadine, a fast dissolving excipient based on co-processed saccharides or polyols having a nonfilamentous microstructure, silicified microcrystalline cellulose and optionally one or more pharmaceutically acceptable excipients.

The water soluble granules may comprise in addition to the active substance one or more pharmaceutically acceptable excipients selected from the group consisting of diluent, e.g. anhydrous lactose, binder, e.g. polyvinylpyrrolidone, assistant binder, disintegrant, super disintegrant, e.g. cross-linked sodium carboxymethylcellulose, acidifying agent, e.g. citric acid, surface active ingredient, e.g. polysorbate, and others known in the art.

A process for the preparation of pharmaceutical composition of the present invention may comprise the following steps:
- preparing the granules comprising active substance,
- blending the granules, fast dissolving excipient based on co-processed saccharides or polyols, silicified microcrystalline cellulose and optional excipients,
- compressing the resulting mixture into tablets by direct compression.

A process for the preparation of pharmaceutical composition of the present invention may comprise the following steps:
- preparing the granules comprising active substance,
- coating the granules,
- blending the coated granules, fast dissolving excipient based on co-processed saccharides or polyols, silicified microcrystalline cellulose and optional excipients,
- compressing the resulting mixture into tablets by direct compression.

The invention is illustrated but not in any way limited by the following examples:

### Example 1:

### Loratadine rapidly disintegrating tablet

Composition:

| | for tbl [mg] | for batch [g] | % |
|---|---|---|---|
| **GRANULATE** | | | |
| loratadine | 10,000 | 214,29 | 7,14% |
| Aerosil 200 | 0,120 | 2,57 | 0,09% |
| citric acid | 0,750 | 16,07 | 0,54% |
| Ac-Di-Sol | 1,400 | 30,00 | 1,00% |
| PVP K25 | 1,000 | 21,43 | 0,71% |
| corn starch | 5,000 | 107,14 | 3,57% |
| anhydrous lactose NF mesh 200 | 15,000 | 321,43 | 10,71% |
| polysorbate 80 V | 1,500 | 32,14 | 1,07% |

| **DRY BLENDED INGREDIENTS** | | | |
|---|---|---|---|
| fast dissolving excipient based on co-processed mannitol and sorbitol comprising particles having a nonfilamentous microstructure* | 79,072 | 1694,40 | 56,48% |
| ProSolv SMCC 90 | 22,004 | 471,51 | 15,72% |
| citric acid | 0,750 | 16,07 | 0,54% |
| aspartame | 0,500 | 10,71 | 0,36% |
| fragrance sweet orange | 0,650 | 13,93 | 0,46% |
| Aerosil 200 | 0,154 | 3,30 | 0,11% |
| Mg stearate | 2,100 | 45,00 | 1,50% |
| **SUM** | | | |
| | 140,000 | 3000,00 | 100,0% |

| | | | |
|---|---|---|---|
| *as described in WO 03/051338 | | | |

### Process for the preparation:

Loratadine and one part of Aerosil 200 are blended in a high shear mixer at low speed (40 - 50 rpm) for a short time. Following addition of Ac-Di-Sol, PVP K25, corn starch, lactose and citric acid into the high shear mixer 10 minutes blending at 50 - 60 rpm is performed. Homogeneous mixture is granulated in high shear mixer with solution of polysorbate in 80 mL of ethanol. Wet granulate is dried in fluid bed dryer. Half dry granulate is pushed through 1,0 mm sieve and returned in to the fluid bed dryer. When loss on drying reaches approximately 2% the drying is completed. Dry granulate pushed twice through 0,5 mm sieve. A fast dissolving excipient based on co-processed mannitol and sorbitol comprising particles having a nonfilamentous microstructure, Prosolv SMCC 90, citric acid, aspartame and fragrance are blended with dry granulate. Aerosil 200 and magnesium stearate are first sieved through 0,5 mm sieve and thereupon blended with the homogenous mixture of dry granulate and dry additions. From the prepared blend 140 mg tablets are made.

### Example 2:

### Loratadine rapidly disintegrating tablet

Composition:

| | for tbl [mg] | for batch [g] | % |
|---|---|---|---|
| **GRANULATE** | | | |
| loratadine | 10,000 | 214,29 | 7,14% |
| Aerosil 200 | 0,120 | 2,57 | 0,09% |
| citric acid | 1,000 | 21,43 | 0,71 % |
| Ac-Di-Sol | 1,400 | 30,00 | 1,00% |
| PVP K25 | 1,400 | 30,00 | 1,00% |
| corn starch | 5,000 | 107,14 | 3,57% |
| anhydrous lactose NF mesh 200 | 25,000 | 535,71 | 17,86% |
| polysorbate 80 V | 1,500 | 32,14 | 1,07% |
| **DRY BLENDED INGREDIENTS** | | | |
| fast dissolving excipient based on co-processed mannitol and sorbitol comprising particles having a nonfilamentous microstructure* | 62,739 | 1344,41 | 44,81% |
| ProSolv SMCC 90 | 28,687 | 614,72 | 20,49% |
| aspartame | 0,250 | 5,36 | 0,18% |
| fragrance menthol | 0,650 | 13,93 | 0,46% |
| Aerosil 200 | 0,154 | 3,30 | 0,11% |
| Mg stearate | 2,100 | 45,00 | 1,50% |
| **SUM** | | | |
| | 140,000 | 3000,00 | 100,0% |

| | | | |
|---|---|---|---|
| *as described in WO 03/051338 | | | |

### Process for the preparation:

Loratadine and one part of Aerosil 200 are blended in a high shear mixer at low speed (40 - 50 rpm) for a short time. Following addition of Ac-Di-Sol, PVP K25, corn starch, lactose and citric acid into the high shear mixer 10 minutes blending at 50 - 60 rpm is performed. Homogeneous mixture is granulated in high shear mixer with solution of polysorbate in 80 mL of ethanol. Wet granulate is dried in fluid bed dryer. Half dry granulate is pushed through 1,0 mm sieve and returned in to the fluid bed dryer. When loss on drying reaches approximately 2% the drying is completed. Dry granulate pushed twice through 0,5 mm sieve. A fast dissolving excipient based on co-processed mannitol and sorbitol comprising particles having a nonfilamentous microstructure, Prosolv SMCC 90, aspartame and fragrance are blended with dry granulate. Aerosil 200 and magnesium stearate are first sieved through 0,5 mm sieve and thereupon blended with the homogenous mixture of dry granulate and dry additions. From the prepared blend 140 mg tablets are made.

## Claims

1. A rapidly disintegrating pharmaceutical composition comprising:
a) granules comprising at least one active substance,
b) a fast dissolving excipient based on co-processed saccharides or polyols, wherein the excipient particles have a nonfilamentous microstructure,
c) silicified microcrystalline cellulose, and
d) optional excipients.

2. The rapidly disintegrating pharmaceutical composition according to claim 1, wherein the fast dissolving excipient based on co-processed saccharides or polyols is a fast dissolving excipient based on co-processed mannitol and sorbitol.

3. The rapidly disintegrating pharmaceutical composition according to any one of claims 1 to 2, wherein active substance is selected from the group consisting of active substances, which need to be granulated before compressing into pharmaceutical compositions.

4. The rapidly disintegrating pharmaceutical composition according to any one of claims 1 to 3, **characterized in that** the active substance is a poorly soluble in water and/or cohesive compound.

5. The pharmaceutical composition according to any one of claims 1 to 4, wherein the active substance is an antihistaminic agent.

6. The pharmaceutical composition according claim 5, wherein the antihistaminic agent is selected from the group consisting of loratadine and desloratadine.

7. The pharmaceutical composition according claim 5, wherein the antihistaminic agent is loratadine.

8. The rapidly disintegrating pharmaceutical composition according to any one of claims 1 to 7, **characterized in that** the granules are water soluble granules.

9. The pharmaceutical composition according to any one of claims 1 to 8, wherein the granules, in addition to the active substance, comprise one or more pharmaceutically acceptable excipients selected from the group consisting of diluents, binders, assistant binders, disintegrants, super disintegrants, acidifying agents, surface active ingredients and other pharmaceutically acceptable excipients.

10. The pharmaceutical composition according to any one of claims 1 to 9, wherein the optional excipients are selected from the group consisting of lubricants, acidifying agents, sweetening agents, flavours, glidants and other pharmaceutically acceptable excipients.

11. The pharmaceutical composition according to any one of claims 1 to 10 **characterized in that** the composition is obtainable by direct compression of the blend comprising the granules comprising said at least one active substance, said fast dissolving excipient, said silicified microcrystalline cellulose, and said optional excipients.

12. A process for the preparation of a pharmaceutical composition according to any one of claims 1 to 10 comprising:
- preparing the granules comprising said at least one active substance which needs to be granulated before compressing into a pharmaceutical composition,
- blending the granules, said fast dissolving excipient, silicified microcrystalline cellulose and optional excipients, and
- compressing the resulting mixture into the tablets.

13. A process for the preparation of a pharmaceutical composition according to any one of claims 1 to 10 comprising:
- preparing the granules comprising said at least one active substance, which needs to be granulated before compressing into a pharmaceutical composition,
- coating the granules,
- blending the granules, said fast dissolving excipient, silicified microcrystalline cellulose and optional excipients, and
- compressing the resulting mixture into tablets form.

## Patentansprüche

1. Rasch zerfallende pharmazeutische Zusammensetzung, umfassend:
a) Granulate, die wenigstens einen Wirkstoff enthalten,
b) einen schnell löslichen Hilfsstoff auf Basis koprozessierter Saccharide oder Polyole, worin die Hilfsstoffpartikel eine nicht filamentöse Mikrostruktur haben,
c) silicierte mikrokristalline Cellulose und
d) optionale Hilfsstoffe.

2. Rasch zerfallende pharmazeutische Zusammensetzung nach Anspruch 1, worin der schnell lösliche Hilfsstoff auf Basis koprozessierter Saccharide oder Polyole ein schnell löslicher Hilfsstoff auf Basis von koprozessiertem Mannitol und Sorbitol ist.

3. Rasch zerfallende pharmazeutische Zusammensetzung nach einem der Ansprüche 1 bis 2, worin der Wirkstoff ausgewählt ist aus der Gruppe, die besteht aus Wirkstoffen, die vor einer Verpressung zu pharmazeutischen Zusammensetzungen granuliert sein müssen.

4. Rasch zerfallende pharmazeutische Zusammensetzung nach einem der Ansprüche 1 bis 3, **dadurch gekennzeichnet, dass** der Wirkstoff eine in Wasser schlecht lösliche und/oder kohäsive Verbindung ist.

5. Pharmazeutische Zusammensetzung nach einem der Ansprüche 1 bis 4, worin der Wirkstoff ein Anthistaminikum ist.

6. Pharmazeutische Zusammensetzung nach Anspruch 5, worin das Antihistaminikum aus der Gruppe ausgewählt ist, die besteht aus Loratadin und Desloratadin.

7. Pharmazeutische Zusammensetzung nach Anspruch 5, worin das Antihistaminikum Loratadin ist.

8. Rasch zerfallende pharmazeutische Zusammensetzung nach einem der Ansprüche 1 bis 7, **dadurch gekennzeichnet, dass** die Granulate in Wasser lösliche Granulate sind.

9. Pharmazeutische Zusammensetzung nach einem der Ansprüche 1 bis 8, worin die Granulate zusätzlich zum Wirkstoff umfassen ein oder mehr pharmazeutisch akzeptable Hilfsstoffe, die ausgewählt sind aus der Gruppe, die besteht aus Verdünnungsmitteln, Bindemitteln, Hilfsbindemitteln, Zerfallmitteln, Superzerfallmitteln, Ansäuerungsmitteln, grenzflächenaktiven Bestandteilen und sonstigen pharmazeutisch akzeptablen Hilfsstoffen.

10. Pharmazeutische Zusammensetzung nach einem der Ansprüche 1 bis 9, worin die optionalen Hilfsstoffe ausgewählt sind aus der Gruppe, die besteht aus Schmiermitteln, Ansäuerungsmitteln, Süßungsmitteln, Aromen, Gleitmitteln und sonstigen pharmazeutisch akzeptablen Hilfsstoffen.

11. Pharmazeutische Zusammensetzung nach einem der Ansprüche 1 bis 10, **dadurch gekennzeichnet, dass** diese Zusammensetzung erhältlich ist durch direkte Verpressung des die Granulate enthaltenden Gemisches, das umfasst wenigstens einen Wirkstoff, den schnell löslichen Hilfsstoff, die silicierte mikrokristalline Cellulose und die optionalen Hilfsstoffe.

12. Verfahren zur Herstellung einer pharmazeutischen Zusammensetzung nach einem der Ansprüche 1 bis 10, umfassend eine
- Herstellung der Granulate, die wenigstens einen Wirkstoff enthalten, der vor einer Kompression zu einer pharmazeutischen Zusammensetzung granuliert werden muss,
- Vermischung der Granulate, des schnell löslichen Hilfsstoffs, der silicierten mikrokristallinen Cellulose und der optionalen Hilfsstoffe, und
- Verpressung des erhaltenen Gemisches zu Tabletten.

13. Verfahren zur Herstellung einer pharmazeutischen Zusammensetzung nach einem der Ansprüche 1 bis 10, umfassend eine
- Herstellung der Granulate, die wenigstens einen Wirkstoff enthalten, der vor einer Verpressung zu einer pharmazeutischen Zusammensetzung granuliert werden muss,
- Beschichtung der Granulate,
- Verpressung der Granulate, des schnell löslichen Hilfsstoffs, der silicierten mikrokristallinen Cellulose und der optionalen Hilfsstoffe, und
- Verpressung des erhaltenen Gemisches zu Tablettenformen.

## Revendications

1. Composition pharmaceutique à désintégration rapide, comprenant :
a) des granules comprenant au moins une substance active,
b) un excipient à dissolution rapide à base de saccharides ou de polyols traités conjointement, les particules d'excipient ayant une microstructure non filamenteuse,
c) de la cellulose microcristalline silicifiée, et
d) des excipients facultatifs.

2. Composition pharmaceutique à désintégration rapide selon la revendication 1, dans laquelle l'excipient à dissolution rapide à base de saccharides ou de polyols traités conjointement est un excipient à dissolution rapide à base de mannitol et de sorbitol traités conjointement.

3. Composition pharmaceutique à désintégration rapide selon l'une quelconque des revendications 1 et 2, dans laquelle la substance active est choisie dans le groupe constitué par des substances actives qui doivent être granulées avant la compression sous forme de compositions pharmaceutiques.

4. Composition pharmaceutique à désintégration rapide selon l'une quelconque des revendications 1 à 3, **caractérisée par le fait que** la substance active est un composé médiocrement soluble dans l'eau et/ou cohésif.

5. Composition pharmaceutique selon l'une quelconque des revendications 1 à 4, dans laquelle la substance active est un agent antihistaminique.

6. Composition pharmaceutique selon la revendication 5, dans laquelle l'agent antihistaminique est choisi dans le groupe constitué par la loratadine et la desloratadine.

7. Composition pharmaceutique selon la revendication 5, dans laquelle l'agent antihistaminique est la loratadine.

8. Composition pharmaceutique à désintégration rapide selon l'une quelconque des revendications 1 à 7, **caractérisée par le fait que** les granules sont des granules hydrosolubles.

9. Composition pharmaceutique selon l'une quelconque des revendications 1 à 8, dans laquelle les granules comprennent, en plus de la substance active, un ou plusieurs excipients pharmaceutiquement acceptables choisis dans le groupe constitué par des diluants, des liants, des liants auxiliaires, des désintégrants, des super-désintégrants, des agents acidifiants, des ingrédients tensioactifs et d'autres excipients pharmaceutiquement acceptables.

10. Composition pharmaceutique selon l'une quelconque des revendications 1 à 9, dans laquelle les excipients facultatifs sont choisis dans le groupe constitué par des lubrifiants, des agents acidifiants, des agents édulcorants, des arômes, des agents de glissement et d'autres excipients pharmaceutiquement acceptables.

11. Composition pharmaceutique selon l'une quelconque des revendications 1 à 10, **caractérisée par le fait que** la composition peut être obtenue par compression directe du mélange comprenant les granules comprenant ladite au moins une substance active, ledit excipient à dissolution rapide, ladite cellulose microcristalline silicifiée et lesdits excipients facultatifs.

12. Procédé de préparation d'une composition pharmaceutique selon l'une quelconque des revendications 1 à 10, comprenant :
- la préparation des granules comprenant ladite au moins une substance active qui doit être granulée avant la compression sous forme d'une composition pharmaceutique,
- le mélange des granules, dudit excipient à dissolution rapide, de la cellulose microcristalline silicifiée et des excipients facultatifs, et
- la compression du mélange résultant sous forme de comprimés.

13. Procédé de préparation d'une composition pharmaceutique selon l'une quelconque des revendications 1 à 10, comprenant :
- la préparation des granules comprenant ladite au moins une substance active qui doit être granulée avant la compression sous forme d'une composition pharmaceutique,
- l'enrobage des granules,
- le mélange des granules, dudit excipient à dissolution rapide, de la cellulose microcristalline silicifiée et des excipients facultatifs, et
- la compression du mélange résultant sous forme de comprimés.
